# EUROPÄISCHE PATENTSCHRIFT

(11) **EP 0 644 719 B1**
(45) Veröffentlichungstag und Bekanntmachung des Hinweises auf die Patenterteilung: **29.12.1997**
(21) Anmeldenummer: 93912555.5
(22) Anmeldetag: 03.06.1993
(51) Int. Cl.: A23F 3/16, A23F 3/32

(54) **WASSERLÖSLICHES INSTANTTEE-ERZEUGNIS**
WATER-SOLUBLE, INSTANT TEA PRODUCT
THE INSTANTANE SOLUBLE DANS L'EAU

(30) Priorität: 10.06.1992 DE 4219330
(43) Veröffentlichungstag der Anmeldung: 29.03.1995
(73) Patentinhaber: Stephan, Günter, D-70469 Stuttgart (DE)
(72) Erfinder: STEPHAN, Günter, D-70469 Stuttgart (DE); STEPHAN, Dieter, D-70469 Stuttgart (DE)
(74) Vertreter: Patentanwälte Wenzel & Kalkoff
(86) Internationale Anmeldenummer: DE9300477
(87) Internationale Veröffentlichungsnummer: WO9325085

(56) Entgegenhaltungen:
- EP-A- 0 464 274
- DE-A- 3 008 836
- FR-A- 2 092 254
- DATABASE WPI Week 8618, 1986 Derwent Publications Ltd., London, GB; AN 86-117167
- Chemisches Zentrallblatt N 19,1960 Seiten 6539-6360

## Beschreibung

Die Erfindung betrifft ein wasserlösliches Instanttee-Erzeugnis in Form eines von einer Trägersubstanz aufgenommenen Tee-Pflanzenextraktes.

Instanttee-Zubereitungen dieser Art sind heute in aller Regel in Pulverform bekannt (DE-A1 32 37 077) sowie verfügbar und aus verschiedenen Gründen sehr gefragt und beliebt; dies besonders deshalb, weil sie einmal eine einfache Zubereitungsform für Tees darstellen und man zum anderen mit Extrakten arbeiten kann, bei denen die üblicherweise in vielen herkömmlichen Teedrogen vorhandenen Rückstände an Pflanzenschutzmitteln, Schwermetallen und/oder radioaktiven Nukleiden durch die Extraktion beseitigt werden können. Bei der Herstellung solcher Instanttee-Zubereitungen werden in der Regel der Tee-Pflanzenextrakt und die ätherischen Öle mit einer aus z.B. Gelatine gewonnenen oder auch anderen geeigneten Trägersubstanz versprüht und so in eine stabile (Pulver-)Form gebracht. Obwohl für Teezubereitungen nur relativ geringe Mengen an pflanzlichem Extrakt und ätherischen Ölen benötigt werden, erhält bzw. benötigt man infolge der Versprühung mit der Trägersubstanz für eine solche Zubereitung eine relativ große Menge an Pulver. Daraus resultieren neben hohen Kosten für die löslichen Eiweiße hohe Verpackungskosten, da normalerweise solche Tees in Glas oder Metalldosen, zudem in verschweißter Form, abgepackt werden. Dabei ergeben sich zudem Probleme aus dem stark hygroskopischen Charakter solcher Instanttees, weil diese Eigenschaft bei einmal angebrochenen Packungen infolge der Feuchtigkeitsaufnahme allein schon während der Benutzung und gesteigert durch nicht absolut dichten Wiederverschluß zum Verkleben bzw. zur Klumpenbildung und damit zur relativ schnellen Unbrauchbarkeit führt. Darüber hinaus wird eine Vielzahl der heutigen Tees an Maltodextrine als Träger gesprüht, was den Nachteil hat, daß solche Tees in erheblichem Maße Zucker enthalten. Dies ist aus den verschiedensten Gründen (Diabetes, Übergewicht u.ä.) unerwünscht.

Weiterhin ist es bekannt (DE-A1 30 08 836), Extrakte, die Kaffee, Tee, Kakao, Suppen od. dgl. sein können, für die Zubereitung von im flüssigen Zustand zu verzehrenden Genuß- und/oder Lebensmitteln portionsweise in wasserlöslichen, als geschmacksneutrales Lebensmittel ausgebildeten Umhüllungen (Kapseln) aufzunehmen und zur Verfügung zu stellen. Solche Umhüllungen können u.a. Gelatine sein. Mit den üblichen Extrakten, wie sie aus den vorbeschriebenen Instantee-Zubereitungen (DE-A1 32 37 077) bekannt sind, würden solche Portionskapseln Größenordnungen annehmen, die einen praktischen Einsatz unzweckmäßig, insbesondere unrentabel machen.

Ein weiteres Produkt zur Bereitung von Tee ist aus der FR-2.092.254 bekannt. Dieses Produkt besteht aus einer zuckrigen Teekonzentratlösung, das von einer Umhüllung umgeben ist, die vorzugsweise aus Zucker besteht. Zur Wahl des Lösungsmittels sind keine Angaben gemacht. Auch hier enthalten die entsprechenden Tees in erheblichem Maße Zucker, was die bereits oben angeführten Nachteile mit sich bringt.

Aus der EP 0 464 274 ist schließlich eine Weichgelatinekapsel mit einem in einem flüssigen Träger enthaltenen, konzentrierten Pflanzenextrakt bekannt, wobei der Träger ein polykondensierter Alkohol, ein Polyol oder oder deren Monomethyl- oder Monoethylether sein kann. Derartige Kapseln finden Anwendung in der Pharmazie zur Einkapselung von für die orale Einnahme bestimmten Medikamenten, wobei sich die Kapsel erst im menschlichen Körper auflösen soll.

Eine Schwierigkeit bei der Herstellung von Tee enthaltenden Kapseln ist, daß die Kapsel selbst zunächst stabil, aber auch wasserlöslich sein muß. Daher wurde bisher ein trockenes Konzentrat eingesetzt, wie dies in der DE-A1 30 08 836 verwirklicht ist. Problematisch hierbei ist jedoch, wie oben ausgeführt, die ausreichende Konzentration des Konzentrats. Um die Substanzmenge unterzubringen, sind relativ große Kapseln erforderlich, die für den Gebrauch unpraktisch und daher wenig akzeptabel sind, d.h. die Anforderung an eine zweckmäßige Darreichungs- und/oder Raumform nicht erfüllen.

Eine weitere Möglichkeit besteht darin, die Substanz in Lösung einzukapseln. Hierbei ist die Wahl des Lösungsmittels kritisch. So scheiden Öle der verschiedensten Form aus, da sie bei der Auflösung in Wasser zu "Fettaugen" auf dem Getränk führen, was vom Verbraucher nicht akzeptiert würde.

Der Erfindung liegt daher die Aufgabe zugrunde, ein wasserlösliches Instanttee-Erzeugnis zur Verfügung zu stellen, das die vorstehend beschriebenen Nachteile beseitigt, insbesondere eine geeignet große Konzentration an Tee-Wirkstoff bei gleichzeitiger Verwendung eines für Wirkstoff und Kapsel geeigneten Lösungsmittels ermöglicht.

Diese Aufgabe wird erfindungsgemäß durch die Verwendung eines von einem wasserlöslichen, leicht verkapselbaren, ölfreien, Lösungs- und Trägermittel aufgenommenen, in einer wasserlöslichen Kapsel verkapselten, als Spissumextrakt hergestellten Tee-Pflanzenextrakts als wasserlösliches zuckerfreies Instanttee-Erzeugnis gelöst.

So können in Lösung der Aufgabenstellung auf kleinstem Raum ohne übermäßige Mengen an Hilfsstoffen die Pflanzlichen Wirkstoffe in einer leicht verpackbaren und manipulierbaren Form in exakt abgemessener Dosis untergebracht und zur Verfügung gestellt werden, wobei für dieses verkapselte Produkt einfachste Verpackungen ohne großen Zusatzaufwand verwendet werden können. Wenn man beispielsweise bedenkt, daß man für die Zubereitung einer Tasse Tee an herkömmlichem Intanttee, zum Beispiel in Pulverform, einen bis eineinhalb Teelöffel Pulver benötigt, was vergleichsweise einer Kapsel gemäß der Erfindung entspricht, so erkennt man ohne weiteres, daß bei letzterer die Menge des Wirkstoffs, bezogen auf die Extraktmenge relativ hoch ist. Darüber hinaus gehen bei dem verkapselten Produkt aber nicht wie bei der trockenen Instanttee-Darreichungsform flüchtige Bestandteile wie ätherische Öle verloren, so daß sich keine Beeinträchtigung des Geschmacksbereichs einstellt.

Durch die Verwendung eines von einem wasserlöslichen, leicht verkapselbaren, ölfreien Lösungs- und Trägermittel aufgenommenen Spissum-Extraktes ist weiterhin sichergestellt, daß das Produkt auch zur Akzeptanz beim Verbraucher führt, da das Auftreten von "Fettaugen" auf dem bereiteten Getränk vermieden wird.

Das Lösungs- und Trägermittel kann bevorzugt ein mehr- oder höhergliedriges Glyzerin wie Triglyzerin sein, da normales Glyzerin durch Gelatine-Kapselwandungen diffundiert. Aber es bieten sich infolge ihres hydrophilen Verhaltens, also der schnellen Lösbarkeit insbesondere in warmem Wasser, wie es für die Teezubereitung verwendet wird, als besonders geeignet auch Tetraethylenglykol, Polyethylenglykole und/oder Polysorbate an. Ein wesentlicher Vorteil derart hergestellter Kapseln besteht darin, daß man mit relativ wenigen Verfahrensschritten bei der Herstellung auskommt und keine zusätzlichen Hilfsstoffe benötigt.

Statt eines in dem Trägerstoff gelösten oder emulgierten Extraktes, der von einer (Weich-)Gelatinekapselhülle eingeschlossen wird, ist es auch denkbar möglich und in manchen Fällen vorteilhaft, wenn man den Tee-Pflanzenextrakt in das - üblicherweise, wie schon erwähnt, aus einer Gelatinezubereitung bestehende - Kapselmaterial einarbeitet, also eine durchgehende Kompaktkapsel erhält.

Als Kapselmaterial sind besonders geeignet Stoffe wie Agar-Agar, Stärke oder Cellulose, wenn und soweit diese hinreichend schnell löslich sind. Diesen Substanzen können gegebenenfalls Geschmacksverbesserer beigemischt sein für den Fall, daß das Lösungsmittel des Extrakts in gewissem Rahmen einen bitteren Geschmack verursachen sollte, wie dies u.a. bei Polyethylenglykolen oder Tetraethylenglykolen der Fall sein kann. Mit derartigen Stoffen des Kapselmaterials erhält man zum ersten Mal eine praktisch zuckerfreie Instanttee-Zubereitung auf rein pflanzlicher Basis.

Zum noch besseren Verständnis der Erfindung und deren Anwendbarkeit werden in folgenden, jedoch ohne irgendeine Beschränkung des Schutzbereiches der Erfindung, einige konkrete Aufführungsbeispiele der Erfindung wiedergegeben:

### 1. Bronchialtee-Kapsel

Bezogen auf die Einheit einer Kapsel enthält diese eine Füllung aus
a. 60,80 mg Extrakt Lichen islandicus aquos. spiss.
   (Verhältnis Droge : Extrakt 16-17 : 1)
   und
b. 259,20 mg Polyethylenglykol 400 (USP
   XXII/Ph.Hel.VII).

Die Kapselhülle besteht aus einer Zusammensetzung
c. 63,558 - 74,612 mg Gelatine (DAB10)
d. 40,218 - 47,212 mg Glycerol 85 %ig (DAB10)
e. 4,224 - 10,176 mg Wasser, gereingt (DAB10).

Eine Kapsel reicht für die Zubereitung einer normalen Tasse Bronchialtee aus, der eine reizlindende Wirkung auf die Schleimhaut im Mund- und Rachenbereich hat.

### 2. Erkältungstee-Kapsel

Bezogen auf die Einheit einer Kapsel enthält diese eine Füllung aus
a. 93,8 mg Tilia e. flor. spir. spiss.
   (Verhältnis Droge : Extrakt 8 : 1)
b. 120 mg Polysorbat (DAB10)
c. 86,2 mg Mannitol, Aspartame, Aromen; in
   veränderlichen Gewichtsanteilen

Die Kapselhülle besteht aus einer Zusammensetzung
d. 58,262 - 68,394 mg Gelatine (DAB10)
e. 36,866 - 43,278 mg Glycerol 85 %ig (DAB10)
f. 3,872 - 9,328 mg Wasser, gereinigt (DAB10).

Eine Kapsel reicht für die Zubereitung einer normalen Tasse Lindenblütentee aus, der bei Erkältungskrankheiten und damit verbundenem Husten eine lindernde und schweißtreibende Wirkung entfaltet.

Die Vorteile der erfindungsgemäßen Teekapsel bestehen, zusammengefaßt, insbesondere in folgendem:
1. Man benötigt für die Zubereitung gleicher Mengen Tee ein wesentlich geringeres Volumen an fertig zur Verfügung gestelltem Instanttee-Erzeugnis; so benötigt man bei herkömmlichem Instanttee für eine Tasse ungefähr einen bis eineinhalb Teelöffel, während das erfindungsgemäße Kapselprodukt in einer Menge einer Kapsel von ungefähr 3 bis 4 mm Durchmesser eine entsprechende Menge an Teesubstanz in der Größenordnung von 100 bis 120 mg, d.h. eine wesentlich höhere Wirkstoffkonzentration, enthält.
2. Durch das hermetische Abschließen der Wirkstoffe in der Kapsel ergeben sich keinerlei Haltbarkeitsprobleme, so daß auf jegliche Konservierungsmittel verzichtet werden kann. Damit können die Verpackungen kleiner und einfacher gestaltet werden, was unter den Aspekten moderner Verpackungsentwicklungen von erheblichem Vorteil ist. Es ergibt sich die Möglichkeit einer Eindosisverpackung, und der Verbraucher kann sich bequem einzelne Dosen, zum Beispiel eine Tagesration von zwei, drei oder mehr Kapseln, mitnehmen.
3. Durch das - bezogen auf eine Verbrauchseinheit, d.h. zum Beispiel eine Tasse Tee - bedingte geringere Volumen und die einfachen, preiswerten Hilfsstoffe, die zum Einsatz gelangen können, erlangt man deutliche Preisvorteile gegenüber Instantteepulver, dessen lösliche Eiweiße sehr teuer sind, abgesehen von der Unverträglichkeit letzterer für manche Menschen.
4. Der Kapselfüllung wie auch dem Kapselmaterial selbst können Geschmacks- und Geruchskorrigenzien zugesetzt werden, die infolge der Verkapselung praktisch unbegrenzt haltbar sind.
5. Durch die erfindungsgemäß vorgeschlagene bevorzugte Wahl von Träger- und/oder Lösungsmittel wird der eingeengte Auszug, der in der Regel eine zähflüssig-klebrige bzw. "schmierige" Substanz darstellt, in geeigneter Weise verarbeitbar bzw. manipulierbar gemacht.

## Patentansprüche

1. Verwendung eines von einem wasserlöslichen, leicht verkapselbaren, ölfreien, Lösungs- und Trägermittel aufgenommenen, in einer wasserlöslichen Kapsel verkapselten, als Spissumextrakt hergestellten Tee-Pflanzenextrakts als wasserlösliches zuckerfreies Instanttee-Erzeugnis.

2. Verwendung nach Anspruch 1, **dadurch gekennzeichnet,** daß das Lösungs- und Trägermittel ein mehr- oder höhergliedriges Glyzerin wie Triglyzerin ist oder aus der Gruppe Tetraäthylenglykol, oder Polyäthylenglykole oder der Polysorbate ausgewählt ist.

3. Verwendung nach Anspruch 1, **dadurch gekennzeichnet,** daß der Tee-Pflanzenextrakt in das aus einer Gelatinezubereitung bestehende Kapselmaterial eingearbeitet ist.

4. Verwendung nach Anspruch 1 oder 2, **dadurch gekennzeichnet,** daß das Material der Kapsel Gelatine, Agar-Agar, Stärke oder Cellulose ist.

## Claims

1. Use of a tea plant extract that is contained in a water soluble, easily capsulisable, oil free solvent and carrier substance, encapsulated in a water soluble capsule and produced as a highly viscous (spissum) extract, as a water soluble sugar free instant tea product.

2. Use according to claim 1, **characterized in** that the solvent and carrier substance is a oligomeric or polymeric glycerol such as triglycerol or is chosen from one of the group consisting of tetraethylene glycol, the polyethylene glycoles or of the polysorbates.

3. Use according to claim 1, **characterized in** that the tea plant extract is incorporated in the capsule material which consists of a gelatin preparation.

4. Use according to claim 1 or 2, **characterized in** that the material of the capsule is gelatin, agar agar, starch or cellulose.

## Revendications

1. Utilisation d'un extrait de thé, préparé sous forme d'un extrait concentré (spissum), encapsulé dans une capsule soluble dans l'eau, repris dans un solvant et support sans huile, facilement encapsulable et soluble dans l'eau, en tant que produit de thé instantané sans sucre et soluble dans l'eau.

2. Utilisation selon la revendication 1, caractérisée en ce que le solvant et support est un polyglycérol ou un glycérol supérieur tel que le triglycérol, ou encore est choisi parmi l'ensemble comprenant le tétraéthylèneglycol, ou les polyéthylèneglycols, ou les polysorbates.

3. Utilisation selon la revendication 1, caractérisée en ce que l'extrait de thé est incorporé dans le matériau de capsule, constitué d'une préparation à base de gélatine.

4. Utilisation selon la revendication 1 ou 2, caractérisé en ce que le matériau de la capsule est la gélatine, l'agar-agar, l'amidon ou la cellulose.
